(19) Europäisches Patentamt
European Patent Office
Office européen des brevets

(11) Veröffentlichungsnummer: **0 050 703**
**B1**

# (12) EUROPÄISCHE PATENTSCHRIFT

(45) Veröffentlichungstag der Patentschrift: **10.04.85**

(51) Int. Cl.⁴: **C 07 H 13/04,** C 07 H 15/04, C 07 H 15/18, A 61 K 31/70 // C07K5/06

(21) Anmeldenummer: **81102907.3**

(22) Anmeldetag: **21.02.79**

(60) Veröffentlichungsnummer der früheren Anmeldung nach Art. 76 EPÜ:

## (54) Neue Glucosederivate und Verfahren zu ihrer Herstellung.

(30) Priorität: **24.02.78 CH 2035/78**
**07.04.78 CH 3777/78**
**18.05.78 CH 5394/78**

(43) Veröffentlichungstag der Anmeldung: **05.05.82 Patentblatt 82/18**

(45) Bekanntmachung des Hinweises auf die Patenterteilung: **10.04.85 Patentblatt 85/15**

(84) Benannte Vertragsstaaten: **BE CH DE FR GB IT LU NL SE**

(56) Entgegenhaltungen:
**FR - A - 2 343 484**
**FR - A - 2 355 505**
**FR - A - 2 358 159**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Baschang, Gerhard, Dr., Bückenweg 7, CH-4126 Bettingen (CH)**
Erfinder: **Dietrich, Felix M., Prof. Dr., Im Nonnengärtli 18, CH-4102 Binningen (CH)**
Erfinder: **Gisler, Roland, Dr., Ob d. Hügliacker 15, CH-4102 Binningen (CH)**
Erfinder: **Hartmann, Albert, Dr., Steingasse 21A, D-7889 Grenzach (DE)**
Erfinder: **Stanek, Jaroslav, Dr., Florastrasse 6, CH-4127 Birsfelden (CH)**
Erfinder: **Tarcsay, Lajos, Dr., Muttenzerstrasse 25, D-7889 Grenzach-Wyhlen (DE)**

(74) Vertreter: **Zumstein, Fritz sen., Dr. et al, Bräuhausstrasse 4, D-8000 München 2 (DE)**

EP 0 050 703 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft N-alkylierte Muramylpeptide und deren Salze sowie Verfahren zu ihrer Herstellung.

Die Erfindung betrifft insbesondere N-alkylierte Muramylpeptide der Formel I

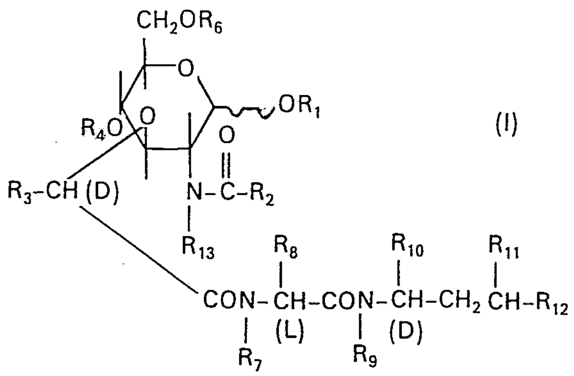

(I)

worin $R_1$ Wasserstoff, Alkyl, gegebenenfalls substituiertes Benzyl oder Acyl, $R_2$ gegebenenfalls substituiertes Alkyl oder carbocyclisches Aryl, $R_4$ und $R_6$ unabhängig voneinander Wasserstoff, Alkyl, gegebenenfalls substituiertes Benzyl oder Acyl, $R_3$ Wasserstoff oder Alkyl, mindestens einer der Reste $R_7$, $R_9$ und $R_{13}$ Niederalkyl, in erster Linie Methyl, und die anderen Wasserstoff, $R_8$ Wasserstoff, Niederalkyl, freies, verestertes oder veräthertes Hydroxyniederalkyl, freies, verestertes oder veräthertes Mercaptoniederalkyl, freies oder acyliertes Aminoniederalkyl, Cycloalkyl mit 5 oder 6 Kohlenstoffatomen, Cycloalkylniederalkyl, dessen Cycloalkylrest 5 oder 6 Kohlenstoffatome enthält, gegebenenfalls substituiertes Aryl oder Aralkyl, stickstoffhaltiges Heterocyclyl oder Heterocyclylniederalkyl, $R_7$ und $R_8$ zusammen auch Alkylen mit 3 oder 4 Kohlenstoffatomen, und die Reste $R_{10}$, $R_{11}$ und $R_{12}$ unabhängig voneinander einen gegebenenfalls veresterten oder amidierten Carboxyrest und $R_{11}$ auch Wasserstoff bedeuten und Salze von solchen Verbindungen mit salzbildenden Gruppen.

Die Konfiguration von Verbindungen, in denen $R_3$, $R_8$ und/oder $R_{10}$ von Wasserstoff verschieden sind, ist in Formel I mit (D) bzw. (L) an den betreffenden Asymmetriezentren angegeben.

Die Bezeichnung «Muramylpeptid» steht streng genommen nur für von der Muraminsäure abgeleitete Verbindungen, in denen $R_3$ Methyl bedeutet. Jedoch werden, falls im Zusammenhang nicht ausdrücklich differenziert, in dieser Anmeldung auch Verbindungen als «Muramylpeptide» bezeichnet, in denen $R_3$ für Wasserstoff oder einen von Methyl verschiedenen Alkylrest steht, und die streng genommen als Normuramyl- bzw. Homomuramylpeptidderivate bezeichnet werden müssten.

Alkyl ist geradkettiges oder verzweigtes, in beliebiger Stellung gebundenes Alkyl mit bis zu 18 Kohlenstoffatomen, in erster Llnie jedoch Niederalkyl.

Als Substituenten der gegebenenfalls substituierten Alkylgruppe kommen in erster LInie freie oder funktionell abgewandelte Hydroxy- oder Mercaptogruppen, wie verätherte oder veresterte Hydroxy- oder Mercaptogruppen, z.B. Niederalkoxy oder Niederalkylmercaptogruppen, oder Halogenatome oder freie oder funktionell abgewandelte Carboxyl-, wie Niederalkoxycarbonyl- oder Carbamoylgruppen in Frage. Dabei kann der substituierte Alkylrest, wie Niederalkylrest, einen, zwei oder mehrere gleiche oder verschiedene Substituenten, insbesondere freie Hydroxygruppen oder Halogenatome tragen.

Carbocyclische Arylreste sind insbesondere monocyclische, sowie bicyclische Arylreste, in erster Linie Phenyl, aber auch Naphthyl. Sie können gegebenenfalls, z.B. durch Niederalkylgruppen, freies, veräthertes oder verestertes Hydroxy, z.B. Niederalkoxy oder Niederalkylendioxy oder Halogenatome, und/oder Trifluoromethylgruppen, mono-, di- oder polysubstituiert sein.

Aralkyl ist insbesondere Arylniederalkyl, worin Aryl die obengenannte Bedeutung hat. In erster Linie steht Arylniederalkyl für Benzyl oder Phenyläthyl, worin der Phenylkern mono-, di- oder polysubstituiert sein kann.

Gegebenenfalls substituierte Benzylreste sind insbesondere solche Benzylreste, die im aromatischen Kern gegebenenfalls, z.B. durch Niederalkyl, freie, verätherte oder veresterte Hxydroxy- oder Mercaptogruppen, z.B. Niederalkoxy- oder Niederalkylendioxy-, sowie Niederalkylmercapto- oder Trifluormethylgruppen und/oder Halogenatome, mono-, di- oder polysubstituiert sind.

Stickstoffhaltiges Heterocyclyl ist insbesondere der Rest einer 5- oder 6gliedrigen, 1 oder 2 Stickstoffatome im Ring enthaltenden heterocyclischen Verbindung. Er kann ungesättigt oder auch gesättigt sein, und z.B. einen ankondensierten Phenylrest enthalten. Als solche seien z.B. der Pyrrol-, Indan-, Pyridyl- oder Imidazolring genannt.

Eine gegebenenfalls veresterte oder amidierte Carboxylgruppe ist in erster Linie die Carboxylgruppe selbst, oder eine mit einem Niederalkanol veresterte Carboxylgruppe oder auch die Carbamoylgruppe, die am Stickstoffatom unsubstituiert ist oder mit Alkyl, insbesondere Niederalkyl, Aryl, in erster Linie Phenyl, oder Aralkyl, wie Benzyl, mono- oder disubstituiert ist. Die Carbamoylgruppe kann aber auch einen Alkylen-, wie den Tetra- oder Pentamethylenrest tragen.

Eine Carbamoylgruppe $R_{10}$ kann am Stickstoff auch durch die Carbamoylmethylgruppe substituiert sein.

Acyl ist insbesondere ein Acylrest einer organischen Säure, insbesondere einer organischen Carbonsäure. So ist Acyl insbesondere Alkanoyl, vor allem mit 2–18 Kohlenstoffatomen, in erster Linie jedoch Niederalkanoyl, oder auch Aroyl, wie Naphthoyl-1, Naphthoyl-2 und insbesondere Benzoyl oder durch Halogen, Niederalkyl, Niederalkoxy, Trifluormethyl, Hydroxy oder Niederalkanoyloxy substituiertes Benzoyl oder Naphthoyl, oder auch ein Acylrest einer organischen Sulfonsäure, z. B. einer Alkansulfonsäure, insbesondere

einer Niederalkansulfonsäure oder einer Alkansulfonsäure, insbesondere einer gegebenenfalls durch Niederalkyl oder Halogen substituierten Phenylsulfonsäure, wie der Benzolsulfonsäure oder p-Toluolsulfonsäure, sowie Carbamoyl, z. B. unsubstituiertes Carbamoyl, Niederalkylcarbamoyl oder Arylcarbamoyl, wie Methyl- oder Phenylcarbamoyl.

Verestertes oder veräthertes Hydroxy ist insbesondere Niederalkoxy oder Niederacyloxy, wie Niederalkanoyloxy.

Verestertes oder veräthertes Mercapto ist insbesondere Niederalkylmercapto- oder Niederacyl-, wie Niederalkanoylmercapto.

Acyliertes Amino ist insbesondere Niederalkanoylamino oder Carbamoylamino.

Die im Zusammenhang mit der vorliegenden Beschreibung und den Patentansprüchen mit «Nieder» bezeichneten Reste und Verbindungen enthalten vorzugsweise bis und mit 7 und in erster Linie bis und mit 4 Kohlenstoffatome.

Vorstehend, wie nachfolgend können die Allgemeinbegriffe folgende Bedeutung haben:

Niederalkyl ist z.B. n-Propyl, n-Butyl, Isobutyl, sek.-Butyl oder tert.-Butyl, ferner n-Pentyl, n-Hexyl, Isohexyl oder n-Heptyl und in erster Linie Methyl oder Äthyl. In Aryl-, Cycloalkyl- oder Heterocyclylniederalkyl ist der Niederalkylrest insbesondere Methyl oder Äthyl, wobei der Aryl-, Cycloalkyl- oder Heterocyclylrest die obengenannte Bedeutung besitzt.

Niederalkoxy ist z.B. n-Propoxy, n-Butoxy, Isobutoxy, sek.-Butoxy oder tert.-Butoxy und in erster Linie Methoxy oder Äthoxy.

Niederalkylmercapto ist z.B. n-Propyl-, n-Butyl-, Isobutyl-, sek.-Butyl- oder tert.-Butylmercapto und in erster Linie Methylmercapto oder Äthylmercapto.

Niederalkylendioxy ist insbesondere Methylendioxy, Äthylen- oder Propylendioxy.

Halogen steht für Fluor oder Brom, vorzugsweise jedoch für Chlor.

Niederalkanoyl ist insbesondere Propionyl oder Butyryl, in erster Linie jedoch Acetyl.

Die neuen Verbindungen der vorliegenden Erfindung können in Form von Gemischen von Isomeren oder von reinen Isomeren vorliegen.

Besonders hervorzuheben sind Verbindungen der Formel I, worin $R_1$, $R_4$ und $R_6$ Wasserstoff, $R_2$ gegebenenfalls durch Hydroxy oder Methoxy substituiertes Niederalkyl oder gegebenenfalls durch Hydroxy, Methoxy, Methyl, Äthyl oder Halogen substituiertes Phenyl, $R_3$ und $R_9$ Wasserstoff oder Methyl, $R_7$ und $R_{13}$ Niederalkyl oder Wasserstoff, mit der Massgabe, dass mindestens einer der Reste $R_7$, $R_9$ und $R_{13}$ für Niederalkyl steht, $R_8$ Methyl, Äthyl, n-Propyl, i-Propyl, 2-Methylpropyl, Methylmercaptomethyl, Hydroxymethyl, Hydroxyäthyl, Phenyl, Benzyl oder p-Hydroxybenzyl, $R_{10}$, $R_{11}$ und $R_{12}$ Carboxy, Niederalkoxycarbonyl oder Carbamoyl und $R_{11}$ auch Wasserstoff bedeuten. Von diesen Verbindungen seien als bevorzugt diejenigen genannt, worin $R_{13}$ für Wasserstoff steht.

Die in allererster Linie bevorzugten Verbindungen sind die in den Beispielen genannten.

Die Verbindungen der Formel I können nach an sich bekannten Methoden hergetellt werden. Beispielsweise können sie erhalten werden, wenn man in an sich bekannter Weise eine Verbindung der Formel

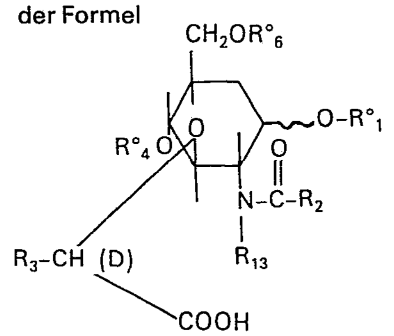

(II)

worin $R_2$, $R_3$ und $R_{13}$ die obengenannte Bedeutung besitzen und $R°_1$, $R°_4$ und $R°_6$ für die Reste $R_1$, $R_4$ bzw. $R_6$ oder für eine leicht abspaltbare Schutzgruppe stehen, oder ein Derivat davon mit einer Verbindung der Formel

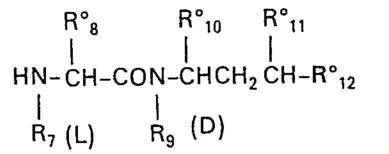

(III)

worin $R_7$ und $R_9$ die obengenannte Bedeutung haben, $R°_8$, $R°_{10}$, $R°_{11}$ und $R°_{12}$ die Bedeutung von $R_8$, $R_{10}$, $R_{11}$ und $R_{12}$ besitzen, mit der Massgabe, dass in diesen Resten vorhandene Carboxy- und, wenn erwünscht, freie Hydroxylgruppen durch leicht abspaltbare Schutzgruppen geschützt sind, kondensiert und gegebenenfalls vorhandene Schutzgruppen abspaltet.

Die Kondensation erfolgt dabei z.B. in der Weise, dass man die Verbindung II in Form der aktivierten Carbonsäure mit der Aminoverbindung III umsetzt oder dass man die Säure II mit der Verbindung III, deren Aminogruppe in aktivierter Form vorliegt, umsetzt. Die aktivierte Carboxylgruppe kann beispielsweise ein Säureanhydrid, vorzugsweise ein gemischtes Säureanhydrid wie ein Säureazid, ein Säureamid, wie ein Imidazolid, Isoxazolid oder ein aktivierter Ester sein. Als aktivierte Ester seien insbesondere genannt: Cyanmethylester, Carboxymethylester, p-Nitrophenylthioester, p-Nitrophenylester, 2,4,5-Trichlorphenylester, Pentachlorphenylester, N-Hydroxysuccinimidester, N-Hydroxyphthalimidester, 8-Hydroxychinolinester, 2-Hydroxy-1,2-dihydro-1-carboäthoxy-chinolin-ester, N-Hydroxypiperidinester oder Enolester, die mit N-Äthyl-5-phenyl-isoxazolium-3'-sulfonat gewonnen werden. Aktivierte Ester können auch gegebenenfalls mit einem Carbodiimid unter Zusatz von N-Hydroxysuccinimid oder einem unsubstituierten oder z.B. durch Halogen, Methyl oder Methoxy substituierten 1-Hydroxy-benzotriazol oder 3-Hydroxy-4-oxo-3,4-dihydro-benzo[d]1,2,3-triazin erhalten werden.

Die Aminogruppe ist beispielsweise durch Reaktion mit einem Phosphitamid aktiviert.

Unter den Methoden der Reaktion mit aktivierten Estern sind insbesondere diejenigen mit N-Äthyl-5-phenyl-isoxazolium-3′-sulfonat (Woodward Reagens K) oder 2-Aethoxy-1,2-dihydro-1-carboäthoxy-chinolin oder Carbodiimid zu erwähnen.

Leicht abspaltbare Schutzgruppen sind solche, die aus der Peptid- bzw. Zuckerchemie bekannt sind. Für Carboxygruppen sollen insbesondere tertiär-Butyl, Benzyl oder Benzhydryl und für Hydroxygruppen insbesondere Acylreste, z.B. Niederalkanoylreste wie Acetyl, Aroylreste, wie Benzoyl, und vor allem von der Kohlensäure sich ableitende Reste, wie Benzyloxycarbonyl oder Niederalkoxycarbonyl, oder Alkyl, insbesondere tert.-Butyl, gegebenenfalls durch Nitro, Niederalkoxy oder Halogen substituiertes Benzyl oder Tetrahydropyranyl oder gegebenenfalls substituierte Alkylidenreste, die die Sauerstoffatome in 4- und 6-Stellung des Glucoseteils verbinden, genannt werden. Solche Alkylidenreste sind insbesondere ein Niederalkyliden-, in erster Linie der Äthyliden-, Isopropyliden- oder Propylidenrest, oder auch ein gegebenenfalls substituierter, vorzugsweise in p-Stellung substituierter Benzylidenrest.

Diese Schutzgruppen können in an sich bekannter Weise abgespalten werden. So kann man sie hydrogenolytisch z.B. mit Wasserstoff in Gegenwart eines Edelmetall-, wie Palladium- oder Platinkatalysators oder durch saure Hydrolyse entfernen.

Die verwendeten Ausgangsstoffe sind bekannt oder lassen sich in an sich bekannter Weise herstellen.

Eine andere Verfahrensweise zur Herstellung dieser neuen Ausgangsstoffe besteht darin, dass man in an sich bekannter Weise eine Verbindung der Formel IV

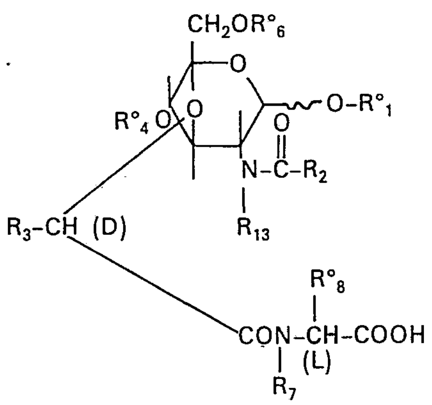

(IV)

worin $R°_1$, $R_2$, $R_3$, $R°_4$, $R°_6$, $R_7$ und $R°_8$ die obengenannte Bedeutung haben, mit einer Verbindung der Formel

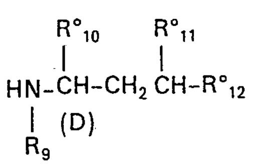

(V)

worin $R_9$, $R°_{10}$, $R°_{11}$ und $R°_{12}$ die obengenannte Bedeutung haben, mit der Massgabe, dass in den Resten $R°_{10}$, $R°_{11}$ und $R°_{12}$ vorhandene Carboxyl- und, wenn erwünscht, freie Hydroxygruppen durch leicht abspaltbare Schutzgruppen geschützt sind, kondensiert und gegebenenfalls vorhandene Schutzgruppen abspaltet.

Die Kondensation erfolgt dabei z.B. in der Weise, dass man die Verbindung IV in Form der aktivierten Carbonsäure mit der Aminoverbindung V umsetzt, oder dass man die Säure IV mit der Verbindung V, deren Aminogruppe in aktivierter Form vorliegt, umsetzt. Die aktivierte Carboxylgruppe kann beispielsweise ein Säureanhydrid, vorzugsweise ein gemischtes Säureanhydrid, ein Säureamid oder ein aktivierter Ester sein. Als solcher kommen insbesondere die obengenannten Säureanhydride, Amide oder Ester in Frage. Die Aminogruppe ist beispielsweise durch Reaktion mit einem Phosphitamid aktiviert.

Auch die leicht abspaltbaren Schutzgruppen entsprechen den bereits oben genannten. Sie können in an sich bekannter Weise abgespalten werden; z.B. hydrogenolytisch, beispielsweise mit Wasserstoff in Gegenwart eines Edelmetall-, wie Palladium- oder Platinkatalysators oder durch saure Hydrolyse.

Die Ausgangsstoffe lassen sich in an sich bekannter Weise erhalten. So kann man z.B. entsprechende in 3-Stellung unsubstituierte Zucker mit einer Halogen-$R_2$-acetamido-$R°_7$-essigsäure umsetzen, oder eine Verbindung der Formel II mit einer Amino-$R_7$-essigsäure, deren Carboxylgruppe geschützt ist, in der oben gezeigten Weise umsetzen, und die Schutzgruppe abspalten.

Eine weitere Verfahrensmethode zur Einführung der in 3-Stellung des Zuckerrestes sitzenden Seitenkette besteht darin, dass man eine Verbindung der Formel

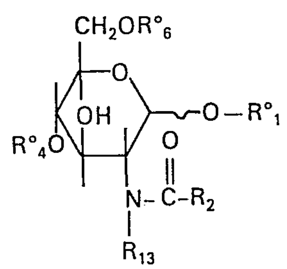

(VI)

worin $R_2$, $R°_1$, $R°_4$, $R°_6$ und $R_{13}$ die obengenannten Bedeutungen haben, und gegebenenfalls darin vorhandene Hydroxygruppen mit einer leicht abspaltbaren Schutzgruppe geschützt sind, mit einer Verbindung der Formel

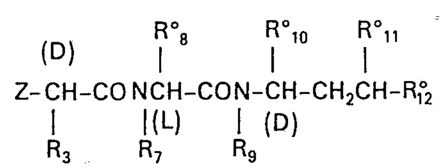

(VII)

umsetzt, worin Z eine reaktionsfähige veresterte Hydroxygruppe darstellt und $R_3$, $R_7$, $R°_8$, $R_9$, $R°_{10}$, $R°_{11}$ und $R°_{12}$ die obengenannte Bedeutung ha-

ben, und gegebenenfalls vorhandene Schutzgruppen abspaltet.

Eine reaktionsfähige veresterte Hydroxygruppe ist insbesondere eine mit einer starken anorganischen oder organischen Säure veresterte Hydroxygruppe, in erster LInie eine solche, die mit einer Halogenwasserstoffsäure, wie Chlor-, Brom- oder Jodwasserstoffsäure, verestert ist.

Die leicht abspaltbaren Schutzgruppen entsprechen den bereits oben genannten. Sie können in an sich bekannter Weise abgespalten werden, z.B. hydrogenolytisch, beispielsweise mit Wasserstoff in Gegenwart eines Edelmetall- wie Palladium- oder Platin-Katalysators, oder durch saure Hydrolyse.

Die erfindungsgemässen Muramylpeptide der Formel I können als Zwischenprodukte zur Herstellung der in der Europäischen Patentanmeldung Nr. 79100513.5 (Publikationsnummer 0 003 833) beschriebenen Muramylpeptid-Antigen-Konjugate verwendet werden.

Die nachstehenden Beispiele illustrieren die Erfindung, ohne sie in irgendeiner Form einzuschränken. Temperaturen sind in Celsiusgraden angegeben.

Beispiel 1

Nach den in dieser Anmeldung beschriebenen Verfahren erhält man 2-Acetylamine-3-0-{[L-1-(D-1-carbamoyl-3-carboxy-propyl)-carbamoyl-äthyl]-N-methyl-carbamoylmethyl}-2-desoxy-D-glucose,

2-Acetylamino-3-0-{[(D-1-carbamoyl-3-carboxy-propyl)-carbamoylmethyl]-N-methyl-carbamoyl-methyl}-2-desoxy-D-glucose,

2-Benzoylamino-3-0-{[(D-1-carbamoyl-3-carboxy-propyl)-carbamoylmethyl]-N-methyl-carbamoylmethyl}-2-desoxy-D-glucose,

2-Acetylamino-3-0-{[(L-1-(D-1-carbamoyl-3-carboxy-propyl)-carbamoyl-äthyl]-N-äthyl-carbamoylmethyl}-2-desoxy-D-glucose,

2-Acetylamino-3-0-{[L-1-(D-1-carbamoyl-3-carboxy-propyl)-carbamoylpropyl]-N-methyl-carbamoylmethyl}-2-desoxy-D-glucose,

2-Acetylamino-3-0-{[L-1-(D-1-carbamoyl-3-carboxy-propyl)-carbamoylpropyl]-N-äthyl-carbamoylmethyl}-2-desoxy-D-glucose

2-Benzoylamino-3-0-{[L-1-(D-1-carbamoyl-3-carboxy-propyl)-carbamoyl-propyl]-N-äthyl-carbamoylmethyl}-2-desoxy-D-glucose,

2-Acetylamino-3-0-{[L-1-(D-1-carbamoyl-3-carboxy-propyl)-carbamoyl-äthyl]-N-propyl-carbamoylmethyl}-2-desoxy-D-glucose,

2-Acetylamino-3-0-{[L-1-(D-1-carbamoyl-3-carboxy-propyl)-N-methyl-carbamoyl-äthyl]-carbamoylmethyl}-2-desoxy-D-glucose,

2-Acetylamino-3-0-{D-1-[(D-1-carbamoyl-3-carboxy-propyl)-carbamoylmethyl]-N-methyl-carbamoyl-äthyl}-2-desoxy-D-glucose,

2-Benzoylamino-3-0-{D-1-[(D-1-carbamoyl-3-carboxy-propyl)-carbamoylmethyl]-N-methyl-carbamoyl-äthyl}-2-desoxy-D-glucose,

2-Acetylamino-3-0-{D-1-[L-1-(D-1-carbamoyl-3-carboxy-propyl)-carbamoyl-äthyl]-N-äthyl-carbamoyl-äthyl}-2-desoxy-D-glucose,

2-Acetylamino-3-0-{D-1-[L-1-(D-1-carbamoyl-3-carboxy-propyl)-carbamoyl-propyl]-N-methyl-carbamoyl-äthyl}-2-desoxy-D-glucose,

2-Acetylamino-3-0-{D-1-[L-1-carbamoyl-3-carboxy-propyl)-carbamoyl-propyl]-N-äthyl-carbamoylmethyl}-2-desoxy-D-glucose,

2-Benzoylamino-3-0-{-D-1-[L-1-(D-1-carbamoyl-3-carboxy-propyl)-carbamoyl-propyl]-N-äthyl-carbamoyl-äthyl}-2-desoxy-D-glucose,

2-Acetylamino-3-0-{D-1-[L-1-(D-1-carbamoyl-3-carboxy-propyl)-carbamoyl-äthyl]-N-propyl-carbamoyl-äthyl}-2-desoxy-D-glucose,

2-Acetylamino-3-0-{D-1-[L-1-(D-1-carbamoyl-3-carboxy-propyl)-N-methyl-carbamoyl-äthyl]-carbamoyl-äthyl}-2-desoxy-D-glucose und

2-Acetylamino-3-0-{[L-1-(D-1,3-dicarboxy-propyl)-N-methyl-carbamoyläthyl]-carbamoylmethyl}-2-desoxy-D-gluocse.

Beispiel 2

Eine Lösung von 4,2 g Benzyl- 2- (N- acetyl- N-methyl)-amino-3-0-{[L-1-(D-1-carbamoyl-3-carboxy- propyl)- carbamoyl- äthyl]- carbamoyl- methyl}- 2- desoxy- D- glucopyranosid in 75 ml Methanol/Wasser 1/1 wird in Gegenwart von 0,5 g 10% Palladium/Kohle bei Normaldruck und 45°C hydriert. Der Katalysator wird abfiltriert und das Filtrat zur Trockne eingedampft. Der Rückstand wird in wenig destilliertem Wasser gelöst und gefriergetrocknet. Man erhält so die 2-(N-Acetyl-N-methyl)-amino-3-0-{[L-1-(D-1-carbamoyl-3-carboxy-propyl)-carbamoyl-äthyl]-carbamoylmethyl}-2-desoxy-D-glucose als weisses Pulver.

Das verwendete Ausgangsmaterial wird wie folgt hergestellt:

Eine Lösung von 8,5 g Benzyl-2-acetylamino-2-desoxy-4,6-0-isopropyliden-3-0-methoxycarbonylmethyl-α-D-glucopyranosid in 80 ml absolutem Acetonitril wird in Stickstoffatmosphäre unter Rühren und Feuchtigkeitsausschluss mit 0,75 g Natriumhydrid (Fluka, pract.) versetzt und 1 Stunde bei 40°C gerührt. Das Reaktionsgemisch wird nun auf Raumtemperatur gekühlt und tropfenweise während 4 Stunden mit einer Lösung von 6,0 g Methyljodid in 50 ml absolutem Acetonitril versetzt. Nach weiteren 3 Stunden wird das Reaktionsgemisch filtriert und das Filtrat zur Trockne eingedampft. Der Rückstand wird in Essigester gelöst, diese Lösung mit Wasser gewaschen, über Natriumsulfat getrocknet und zur Trockne eingedampft. Man erhält so das Benzyl-2-(N-acetyl-N-methyl)-amino-2-desoxy-4,6-0-isopropyliden-3-0-methoxycarbonylmethyl-α-D-glucopyranosid als gelbes Öl, Rf = 0,45 (Methylenchlorid/Essigester = 85/15 auf Kieselgeldünnschichtplatten).

Eine Lösung von 4,4 g Benzyl-2-(N-acetyl-N-methyl)-amino-2-desoxy-4,6-0-isopropyliden-3-0-methoxycarbonylmethyl-α-D-glucopyranosid in 60 ml Methanol und 15 ml 1 n Natronlauge wird 1 Stunde bei Raumtemperatur stehen gelassen, mit 5 ml 1 n Salzsäure versetzt und zur Trockne eingedampft. Das erhaltene Benzyl-2-(N-acetyl-

N-methyl)-amino-3-0-carboxymethyl-2-desoxy-4,6-0-isopropyliden-α-D-glucopyranosid-natriumsalz wird in 50 ml N,N-Dimethylformamid gelöst und mit 3,2 g L-Alanyl-D-isoglutamin-tert.butylester-hydrochlorid in Gegenwart von 2,5 g EEDQ kondensiert. Die Lösung wird im Vakuum zur Trockne eingedampft und der Rückstand in Essigester gelöst. Man wäscht diese Lösung nacheinander mit Wasser, eiskalter 1 n Salzsäure, Wasser, einer gesättigten Natriumhydrogencarbonat-Lösung und Wasser, trocknet über Magnesiumsulfat und dampft zur Trockne ein. Man erhält so Benzyl-2-(N-acetyl-N-methyl)-amino-3-0-{[L-1-(D-1-carbamoyl-3-carboxy-propyl)-carbamoyl-äthyl]-carbamoylmethyl}-2-desoxy-4,6-0-isopropyliden-α-D-glucopyranosid-tert.butylester als gelblichen Schaum. Dieses Produkt wird in 45 ml auf 0°C vorgekühlter 95%iger Trifluoressigsäure gelöst und 1 Stunde bei 0°C gerührt. Das Reaktionsgemisch wird auf 400 ml absoluten Äther gegossen, das ausgefallene Produkt abgesaugt, mit Äther gewaschen und getrocknet. Durch Behandlung dieser Substanz mit dem Ionenaustauscherharz Dowex-3 (Acetat-Form) erhält man das trifluoressigsäurefreie Benzyl-2-(N-acetyl-N-methyl)-amino-3-0-{[L-1-(D-1-carbamoyl-3-carboxy-propyl)-carbamoyl-äthyl]-carbamoyl-methyl}-2-desoxy-α-D-glucopyranosid als weisses Pulver.

Auf analoge Weise werden hergestellt:

2-(N-Acetyl-N-methyl)-amino-3-0-{D-1-[L-1-(D-1-carbamoyl-3-carboxy-propyl)-carbamoyl-äthyl]-carbymoyl-äthyl}-2-desoxy-D-glucose,
2-(N-Acetyl-N-methyl)-amino-3-0-{D-1-[L-1-(D-1-carbamoyl-3-carboxy-propyl)-carbamoyl-propyl]-carbamoyl-äthyl}-2-desoxy-D-glucose,
2-(N-Acetyl-N-methyl)-amino-3-0-{[L-1-(D-1-carbamoyl-3-carboxy-propyl)-carbamoyl-propyl]-carbamoylmethyl}-2-desoxy-D-glucose,
2-(N-Acetyl-N-methyl)-amino-3-0-{[L-1-(D-1-carbamoyl-3-carboxy-propyl)-carbamoyl-2-methyl-propyl]-carbamoylmethyl}-2-desoxy-D-glucose,
2-(N-Acetyl-N-methyl)-amino-3-0-{D-1-[L-1-(D-1-carbamoyl-3-carboxy-propyl)-carbamoyl-2′-methyl-propyl]-carbamoyl-äthyl}-2-desoxy-D-glucose,
3-0-{[L-1-(D-1-Carbamoyl-3-carboxy-propyl)-carbamoyl-äthyl]-carbamoyl-methyl}-2-desoxy-2-(N-propionyl-N-methyl)-amino-D-glucose,
2-(N-Butyryl-N-methyl)-amino-3-0-{[L-1-(D-1-carbamoyl-3-carboxy-propyl)-carbamoyl-äthyl]-carbamoylmethyl}-2-desoxy-D-glucose.

**Patentansprüche**

1. Verbindungen der Formel I

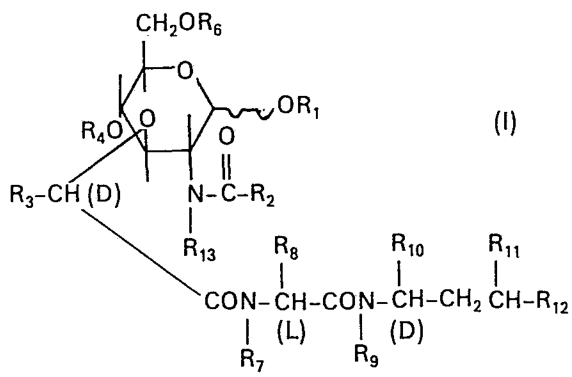

(I)

worin $R_1$ Wasserstoff, Alkyl, gegebenenfalls substituiertes Benzyl oder Acyl, $R_2$ gegebenenfalls substituiertes Alkyl oder carbocyclisches Aryl, $R_4$ und $R_6$ unabhängig vorneinander Wasserstoff, Alkyl, gegebenenfalls substituiertes Benzyl oder Acyl, $R_3$ Wasserstoff oder Alkyl, mindestens einer der Reste $R_7$, $R_9$ und $R_{13}$ Niederalkyl und die anderen Wasserstoff, $R_8$ Wasserstoff, Niederalkyl, freies, verestertes oder veräthertes Hydroxyniederalkyl, freies, verestertes oder veräthertes Mercaptoniederalkyl, freies oder acyliertes Aminoniederalkyl, Cycloalkyl mit 5 oder 6 Kohlenstoffatomen, Cycloalkylniederalkyl, dessen Cycloalkylrest 5 oder 6 Kohlenstoffatome enthält, gegebenenfalls substituiertes Aryl oder Aralkyl, stickstoffhaltiges Heterocyclyl oder Heterocyclylniederalkyl, $R_7$ und $R_8$ zusammen auch Alkylen mit 3 oder 4 Kohlenstoffatomen, und die Reste $R_{10}$, $R_{11}$ und $R_{12}$ unabhängig voneinander einen gegebenenfalls veresterten oder amidierten Carboxyrest und $R_{11}$ auch Wasserstoff bedeuten, wobei das Wort Alkyl einen Alkylrest mit bis zu 18 und das Präfix «Nieder» einen Rest bis und mit 7 C-Atomen kennzeichnet, und Salze von solchen Verbindungen mit salzbildenden Gruppen.

2. Verbindungen der Formel I gemäss Anspruch 1, worin mindestens einer der Reste $R_7$, $R_9$ und $R_{13}$ für Methyl steht und deren Salze.

3. Verbindungen der Formel I nach Anspruch 1 oder 2, worin $R_1$, $R_4$ und $R_6$ Wasserstoff, $R_2$ gegebenenfalls durch Hydroxy oder Methoxy substituiertes Niederalkyl oder gegebenenfalls durch Hydroxy, Methoxy, Methyl, Äthyl oder Halogen substituiertes Phenyl, $R_3$ und $R_9$ Wasserstoff oder Methyl, $R_8$ Methyl, Äthyl, n-Propyl, i-Propyl, 2-Methylpropyl, Methylmercaptomethyl, Hydroxymethyl, Hydroxyäthyl, Phenyl, Benzyl oder p-Hydroxybenzyl, $R_{10}$, $R_{11}$ und $R_{12}$ Carboxy, Niederalkoxycarbonyl oder Carbamoyl oder $R_{11}$ auch Wasserstoff bedeuten, und deren Salze.

4. Verbindungen der Formel I gemäss Anspruch 1, worin $R_1$, $R_4$, $R_6$ und $R_{11}$ Wasserstoff, $R_2$

Methyl oder Phenyl, $R_3$ Wasserstoff oder Methyl, $R_7$ Methyl, Äthyl oder Propyl, $R_8$ Wasserstoff, Methyl oder Äthyl, $R_9$ Wasserstoff oder Methyl, $R_{10}$ Carbamoyl, $R_{12}$ Carboxy und $R_{13}$ Wasserstoff oder Methyl bedeuten, und ihre Salze.

5. Verbindungen der Formel I nach einem der Ansprüche 1–3, worin $R_{13}$ für Wasserstoff steht, und deren Salze.

6. N-Acetyl-normuramyl-L-(N-methyl)-alanyl-D-isoglutamin ≡ 2-Acetyl-amino-3-0-{[L-1(D-1-carbamoyl-3-carboxy-propyl)-carbamoyl-äthyl]-N-methyl-carbamoylmethyl}-2-desoxy-D-glucose und ihre Salze gemäss Anspruch 1.

7. N-Benzoyl-normuramyl-L-(N-äthyl)-α-amino-butyryl-D-isoglutamin ≡ 2-Benzoylamino-3-0-{[L-1-(D-1-carbamoyl-3-carboxy-propyl)-carbamoylpropyl]-N-äthyl-carbamoylmethyl}-2-desoxy-D-glucose und ihre Salze gemäss Anspruch 1.

8. N-Acetyl-muramyl-L-(N-methyl)-α-amino-butyryl-D-isoglutamin ≡ 2-Acetylamino-3-0-{D-1-[L-1-(D-1-carbamoyl-3-carboxy-propyl)-carbamoyl-propyl]-N-methyl-carbamoyl-äthyl}-2-desoxy-D-glucose und ihre Salze gemäss Anspruch 1.

9. N-Acetyl-muramyl-L-(N-äthyl)-alanyl-D-isoglutamin ≡ 2-Acetylamino-3-0-{D-1-[L-1-(D-1-carbamoyl-3-carboxy-propyl)-carbamoyl-äthyl]-N-äthyl-carbamoyl-äthyl}-2-desoxy-D-glucose und ihre Salze gemäss Anspruch 1.

10. Verfahren zur Herstellung von Verbindungen der Formel I

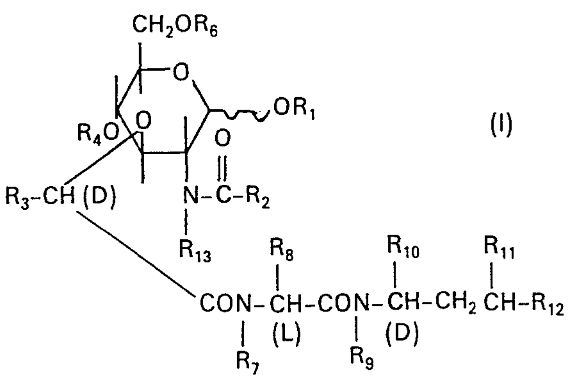

(I)

worin $R_1$ Wasserstoff, Alkyl, gegebenenfalls substituiertes Benzyl oder Acyl, $R_2$ gegebenenfalls substituiertes Alkyl oder carbocyclisches Aryl, $R_4$ und $R_6$ unabhängig voneinander Wasserstoff, Alkyl, gegebenenfalls substituiertes Benzyl oder Acyl, $R_3$ Wasserstoff oder Alkyl, mindestens einer der Reste $R_7$ $R_9$ und $R_{13}$ Niederalkyl und die anderen Wasserstoff, $R_8$ Wasserstoff, Niederalkyl, freies, verestertes oder veräthertes Hydroxyniederalkyl, freies, verestertes oder veräthertes Mercaptoniederalkyl, freies oder acyliertes Aminoniederalkyl, Cycloalkyl mit 5 oder 6 Kohlenstoffatomen, Cycloalkylniederalkyl, dessen Cycloalkylrest 5 oder 6 Kohlenstoffatome enthält, gegebenenfalls substituiertes Aryl oder Aralkyl, stickstoffhaltiges Heterocyclyl oder Heterocyclylniederalkyl, $R_7$ und $R_8$ zusammen auch Alkylen mit 3 oder 4 Kohlenstoffatomen und die Reste $R_{10}$, $R_{11}$ und $R_{12}$ unabhängig voneinander einen gegebenenfalls veresterten oder amidierten Carboxyrest und $R_{11}$ auch Wasserstoff bedeuten, und Salze von solchen Verbindungen mit salzbildenden Gruppen, dadurch gekennzeichnet, dass man in an sich bekannter Weise eine Verbindung der Formel

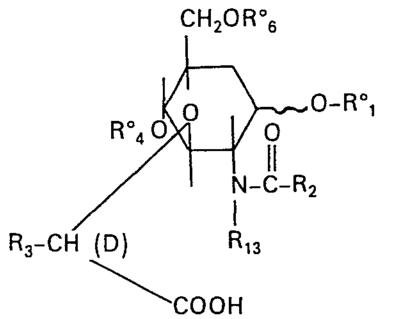

(II)

worin $R_2$, $R_3$ und $R_{13}$ die obengenannte Bedeutung besitzen und $R°_1$, $R°_4$ und $R°_6$ für die Reste $R_1$, $R_4$ bzw. $R_6$ oder für eine leicht abspaltbare Schutzgruppe stehen, oder ein Derivat davon mit einer Verbindung der Formel

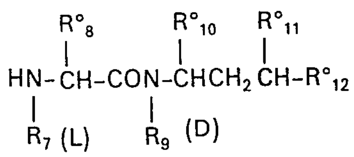

(III)

worin $R_7$ und $R_9$ die obengenannte Bedeutung besitzen und $R°_8$, $R°_{10}$, $R°_{11}$ und $R°_{12}$ die Bedeutung von $R_8$, $R_{10}$, $R_{11}$ und $R_{12}$ besitzen, mit der Massgabe, dass in diesen Resten vorhandene Carboxy- und, wenn erwünscht, freie Hydroxylgruppen durch leicht abspaltbare Schutzgruppen geschützt sind, kondensiert und gegebenenfalls vorhandene Schutzgruppen abspaltet, oder dass man in an sich bekannter Weise eine Verbindung der Formel IV,

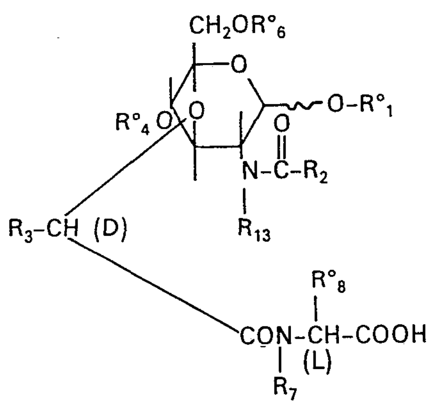

(IV)

worin die Substituenten die obengenannte Bedeutung haben, mit einer Verbindung der Formel

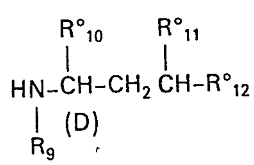

(V)

worin $R_9$, $R°_{10}$, $R°_{11}$ und $R°_{12}$ die obengenannte Bedeutung haben, mit der Massgabe, dass in den Resten $R°_{10}$, $R°_{11}$ und $R°_{12}$ vorhandene Carboxyl- und, wenn erwünscht, freie Hydroxygruppen durch leicht abspaltbare Schutzgruppen geschützt sind, kondensiert und gegebenenfalls vorhandene Schutzgruppen abspaltet, oder dass man eine Verbindung der Formel

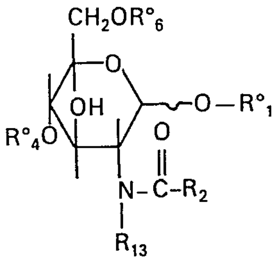

(VI)

worin $R_2$, $R°_1$, $R°_4$, $R°_6$ und $R_{13}$ die obengenannten Bedeutungen haben, und gegebenenfalls darin vorhandene Hydroxygruppen mit einer leicht abspaltbaren Schutzgruppe geschützt sind, mit einer Verbindung der Formel

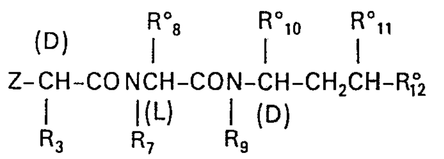

(VII)

umsetzt, worin Z eine reaktionsfähige veresterte Hydroxygruppe darstellt und die anderen Substituenten die oben angegebene Bedeutung haben, und gegebenenfalls vorhandene Schutzgruppen abspaltet, wobei leicht abspaltbare Schutzgruppen solche sind, die aus der Peptid- bzw. Zuckerchemie bekannt sind.

**Claims**

1. A compound of formula I

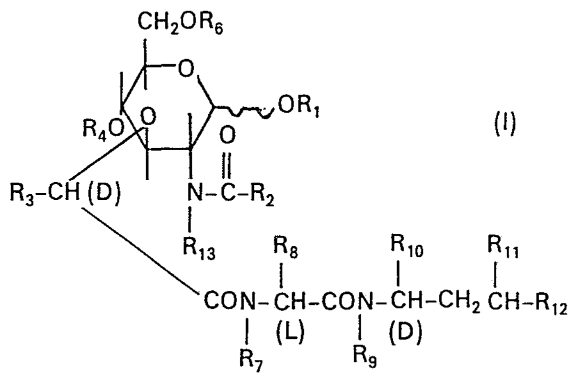

(I)

wherein

$R_1$ is hydrogen, alkyl, unsubstituted or substituted benzyl or acyl, $R_2$ is unsubstituted or substituted alkyl or carbocyclic aryl, $R_4$ and $R_6$ are each independently hydrogen, alkyl, unsubstituted or substituted benzyl or acyl, $R_3$ is hydrogen or alkyl, at least one of $R_7$, $R_9$ and $R_{13}$ is lower alkyl and the others are hydrogen, $R_8$ is hydrogen, lower alkyl, free, esterified or etherified hydroxy-lower alkyl, free, esterified or etherified mercapto-lower alkyl, free or acylated amino-lower alkyl, cycloalkyl containing 5 or 6 carbon atoms, cycloalkyl-lower alkyl, the cycloalkyl radical of which contains 5 or 6 carbon atoms, unsubstituted or substituted aryl or aralkyl, nitrogen-containing heterocyclyl or heterocyclyl-lower alkyl, $R_7$ and $R_8$ together are also alkylene containing 3 or 4 carbon atoms, and each of $R_{10}$, $R_{11}$ and $R_{12}$ independently is a free, esterified or amidated carboxyl group, and $R_{11}$ is also hydrogen, the term 'alkyl' denoting an alkyl radical containing not more than 18 carbon atoms, and where such radical is qualified by the prefix 'lower', it contains up to and including 7 carbon atoms; or a salt of such a compound containing salt-forming groups.

2. A compound of formula I according to claim 1, wherein at least one of $R_7$, $R_9$ and $R_{13}$ is methyl, or a salt thereof.

3. A compound of formula 1 according to either claim 1 or claim 2, wherein $R_1$, $R_4$ and $R_6$ are hydrogen, $R_2$ is lower alkyl or lower alkyl substituted by hydroxy or methoxy, or is phenyl or phenyl substituted by hydroxy, methoxy, methyl, ethyl or halogen, $R_3$ and $R_9$ are hydrogen or methyl, $R_8$ is methyl, ethyl, n-propyl, i-propyl, 2-methylpropyl, methylmercaptomethyl, hydroxymethyl, hydroxyethyl, phenyl, benzyl or p-hydroxybenzyl, $R_{10}$, $R_{11}$ and $R_{12}$ are carboxy, lower alkoxycarbonyl or carbamoyl or $R_{11}$ is also hydrogen, or a salt thereof.

4. A compound of formula I according to claim 1, wherein $R_1$, $R_4$, $R_6$ and $R_{11}$ are hydrogen, $R_2$ is methyl or phenyl, $R_3$ is hydrogen or methyl, $R_7$ is methyl, ethyl or propyl, $R_8$ is hydrogen, methyl or ethyl, $R_9$ is hydrogen or methyl, $R_{10}$ is carbamoyl, $R_{12}$ is carboxy and $R_{13}$ is hydrogen or methyl, or a salt thereof.

5. A compound of formula I according to any one of claims 1 to 3, wherein $R_{13}$ is hydrogen, or a salt thereof.

6. N-acetyl-normuramyl-L-(N-methyl)-alanyl-D-isoglutamine ≡ 2-acetylamino-3-0-{[L-1(D-1-carbamoyl-3-carboxy-propyl)-carbamoyl-ethyl]-N-methyl-carbamoylmethyl}-2-desoxy-D-glucose, or a salt thereof, according to claim 1.

7. N-benzoyl-normuramyl-L-(N-ethyl)-α-amino-butyryl-D-isoglutamine ≡ 2-benzoylamino-3-0-{[L-1-(D-1-carbamoyl-3-carboxy-propyl)-carbamoyl-propyl]-N-ethyl-carbamoylmethyl}-2-desoxy-D-glucose, or a salt thereof, according to claim 1.

8. N-acetyl-muramyl-L-(N-methyl)-α-amino-butyryl-D-isoglutamine ≡ 2-acetylamino-3-0-{D-1-[L-1-(D-1-carbamoyl-3-carboxy-propyl)-carbamoyl-propyl]-N-methyl-carbamoyl-ethyl}-2-desoxy-D-glucose, or a salt thereof, according to claim 1.

9. N-acetyl-muramyl-L-(N-ethyl)-alanyl-D-isoglutamine ≡ 2-acetyl-amino-3-0-{D-1-[L-1-(D-1-carbamoyl-3-carboxy-propyl)-carbamoyl-ethyl]-N-ethyl-carbamoyl-ethyl}-2-desoxy-D-glucose, or a salt thereof, according to claim 1.

10. A process for the preparation of a compound of formula I

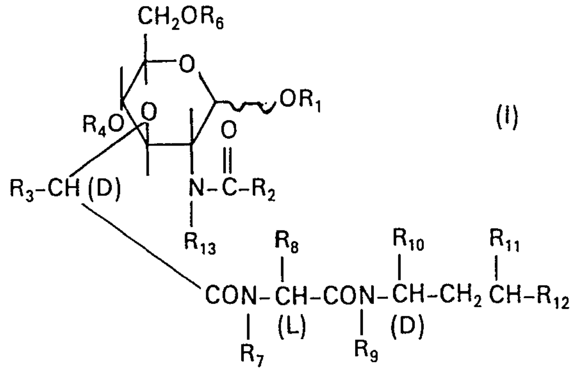

(I)

wherein
$R_1$ is hydrogen, alkyl, unsubstituted or substituted benzyl or acyl, $R_2$ is unsubstituted or substituted alkyl or carbocyclic aryl, $R_4$ and $R_6$ are each independently hydrogen, alkyl, unsubstituted or substituted benzyl or acyl, $R_3$ is hydrogen or alkyl, at least one of $R_7$, $R_9$ and $R_{13}$ is lower alkyl and the others are hydrogen, $R_8$ is hydrogen, lower alkyl, free, esterified or etherified hydroxy-lower alkyl, free, esterified or etherified mercapto-lower alkyl, free or acylated amino-lower alkyl, cycloalkyl containing 5 or 6 carbon atoms, cycloalkyl-lower alkyl, the cycloalkyl radical of which contains 5 or 6 carbon atoms, unsubstituted or substituted aryl or aralkyl, nitrogen-containing heterocyclyl or heterocyclyl-lower alkyl, $R_7$ and $R_8$ together are also alkylene containing 3 or 4 carbon atoms, and each of $R_{10}$, $R_{11}$ and $R_{12}$ independently is a free, esterified or amidated carboxyl group, and $R_{11}$ is also hydrogen, or a salt of such a compound containing salt-forming groups, which process comprises condensing in a manner known per se a compound of the formula

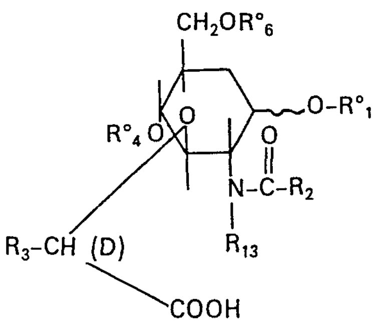

(II)

wherein
$R_2$, $R_3$ and $R_{13}$ are as defined above and $R°_1$, $R°_4$ and $R°_6$ have the same meanings as $R_1$, $R_4$ or $R_6$ or are an easily removable protective group, or a derivative thereof, with a compound of the formula

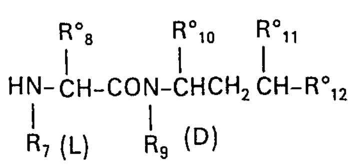

(III)

wherein
$R_7$ and $R_9$ are as defined above and $R°_8$, $R°_{10}$, $R°_{11}$ and $R°_{12}$ have the same meanings as $R_8$, $R_{10}$, $R_{11}$ and $R_{12}$, with the proviso that carboxyl groups and, if desired, free hydroxyl groups present in said radicals are protected by easily removable protective groups, and removing any protective groups present, or condensing in a manner known per se a compound of formula IV

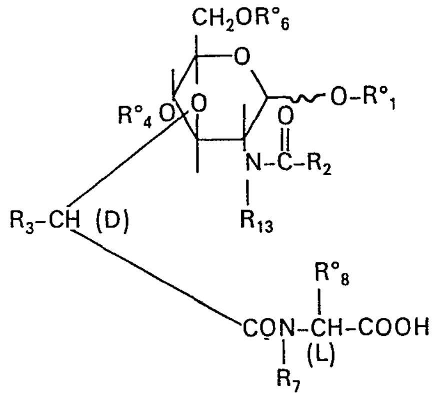

(IV)

wherein
the substituents are as defined above, with a compound of the formula

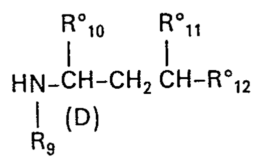

(V)

wherein
$R_9$, $R°_{10}$, $R°_{11}$ and $R°_{12}$ are as defined above, with the proviso that carboxyl groups and, if desired, free hydroxyl groups present in the radicals are protected by easily removable protective groups, and removing any protective groups present, or reacting a compound of the formula

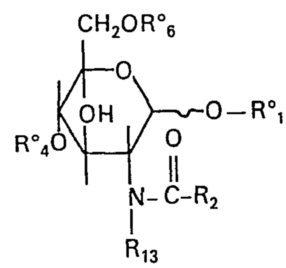

(VI)

wherein
$R_2$, $R°_1$, $R°_4$, $R°_6$ and $R_{13}$ are as defined above and any hydroxyl groups present therein are protected by an easily removable protective group, with a compound of the formula

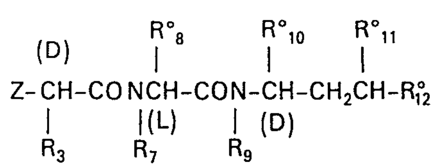

(VII)

wherein
Z is a reactive esterified hydroxyl group and the other substituents are as defined above, and removing any protective groups present, easily removable protective groups being those groups known in peptide or sugar chemistry.

**Revendications**

1. Composés de formule I

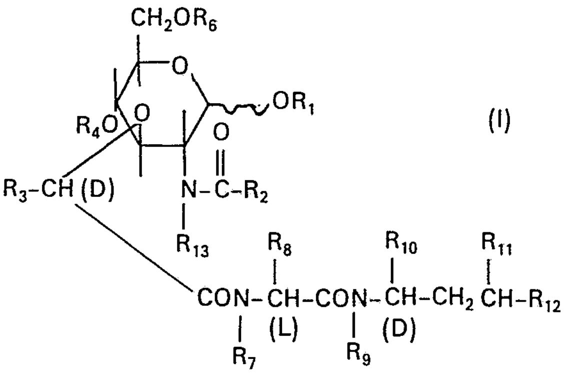

(I)

où $R_1$ représente un hydrogène, un alcoyle, un benzyle ou un acyle éventuellement substitué, $R_2$ représente un alcoyle éventuellement substitué ou un aryle carbocyclique, $R_4$ et $R_6$ représentent indépendamment l'un de l'autre un hydrogène, un alcoyle, un benzyle ou un acyle éventuellement substitué, $R_3$ représente un hydrogène ou un alcoyle, au moins l'un des radicaux $R_7$, $R_9$ et $R_{13}$ représente un alcoyle inférieur et les autres représentent un hydrogène, $R_8$ représente un hydrogène, un alcoyle inférieur, un hydroxyalcoyle inférieur libre, estérifié ou éthérifié, un mercaptoalcoyle inférieur libre, estérifié ou éthérifié, un aminoalcoyle inférieur libre ou acylé, un cycloalcoyle en $C_5$ ou $C_6$, un cycloalcoyle-alcoyle inférieur dont le radical cycloalcoyle contient 5 ou 6 atomes de carbone, un aryle ou un aralcoyle éventuellement substitué, un hétérocyclyle ou un hétérocyclylalcoyle inférieur contenant de l'azote, $R_7$ et $R_8$ représentent également ensemble un alcoylène en $C_3$ ou $C_4$, et les radicaux $R_{10}$, $R_{11}$ et $R_{12}$ représentent indépendamment l'un de l'autre un radical carboxy éventuellement estérifié ou amidé et $R_{11}$ représente également un hydrogène, où le mot «alcoyle» désigne un radical alcoyle ayant jusqu'à 18 et le préfixe «inférieur» un radical ayant jusqu'à 7 atomes de carbone compris, et les sels de tels composés avec des groupes salificateurs.

2. Composés de formule I selon la revendication 1, où au moins un des radicaux $R_7$, $R_9$ et $R_{13}$ représente un méthyle, et leurs sels.

3. Composés de formule I selon les revendications 1 et 2, où $R_1$, $R_4$ et $R_6$ représentent un hydrogène, $R_2$ représente un alcoyle inférieur éventuellement substitué par un hydroxy ou un méthoxy ou un phényle éventuellement substitué par un hydroxy, un méthoxy, un méthyle, un éthyle ou un halogène, $R_3$ et $R_9$ représentent un hydrogène ou un méthyle, $R_8$ représente un méthyle, un éthyle, un n-propyle, un i-propyle, un 2-méthylpropyle, un méthylmercaptométhyle, un hydroxyméthyle, un hydroxyéthyle, un phényle, un benzyle ou un p-hydroxybenzyle, $R_{10}$, $R_{11}$ et $R_{12}$ représentent un carboxy, un alcoxy inférieur-carbonyle ou un carbamoyle ou $R_{11}$ représente également un hydrogène, et leurs sels.

4. Composés de formule I selon la revendication 1, où $R_1$, $R_4$, $R_6$ et $R_{11}$ représentent un hydrogène, $R_2$ représente un méthyle ou un phényle, $R_3$ représente un hydrogène ou un méthyle, $R_7$ représente un méthyle, un éthyle ou un propyle, $R_8$ représente un hydrogène, un méthyle ou un éthyle, $R_9$ représente un hydrogène ou un méthyle, $R_{10}$ représente un carbamoyle, $R_{12}$ représente un carboxy et $R_{13}$ représente un hydrogène ou un méthyle, et leurs sels.

5. Composés de formule I selon l'une des revendications 1–3, où $R_{13}$ représente un hydrogène, et leurs sels.

6. N-acétyl-normuramyl-L-(N-méthyl)-alanyl-D-isoglutamine ≡ 2-acétyl-amino-3-0-{[L-1(D-1-carbamoyl-3-carboxypropyl)-carbamoyl-éthyl]-N-méthyl-carbamoylméthyl}-2-désoxy-D-glucose et ses sels selon la revendication 1.

7. N-benzoyl-normuramyl-L-(N-éthyl)-α-amino-butyryl-D-isoglutamine ≡ 2-benzoylamino-3-0-{[L-1-(D-1-carbamoyl-3-carboxy-propyl)-carbamoyl-propyl]-N-éthyl-carbamoyl-méthyl}-2-désoxy-D-glucose et ses sels selon la revendication 1.

8. N-acétyl-muramyl-L-(N-méthyl)-α-amino-butyryl-D-isoglutamine ≡ 2-acétylamino-3-0-{D-1-[L-1-(D-1-carbamoyl-3-carboxy-propyl)-carabamoyl-propyl]-N-méthyl-carbamoyl-éthyl}-2-désoxy-D-glucose et ses sels selon la revendication 1.

9. N-acétyl-muramyl-L-(N-éthyl)-alanyl-D-isoglutamine ≡ 2-acétylamino-3-0-{D-1-[L-1-(D-1-carbamoyl-3-carboxy-propyl)-carbamoyl-éthyl]-N-éthyl-carbamoyl-éthyl}-2-désoxy-D-glucose et ses sels selon la revendication 1.

10. Procédé de préparation de composés de formule I

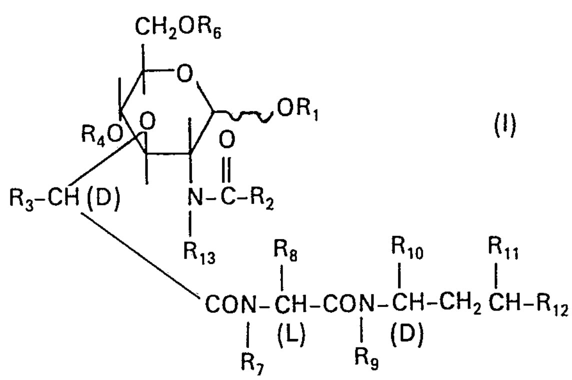

(I)

où $R_1$ représente un hydrogène, un alcoyle, un benzyle ou un acyle éventuellement substitué, $R_2$ représente un alcoyle éventuellement substitué ou un aryle carbocyclique, $R_4$ et $R_6$ représentent indépendamment l'un de l'autre un hydrogène, un alcoyle, un benzyle ou un acyle éventuelle-

ment substitué, $R_3$ représente un hydrogène ou un alcoyle, au moins l'un des radicaux $R_7$, $R_9$ et $R_{13}$ représente un alcoyle inférieur et les autres représentent un hydrogène, $R_8$ représente un hydrogène, un alcoyle inférieur, un hydroxyalcoyle inférieur libre, estérifié ou éthérifié, un mercaptoalcoyle inférieur libre, estérifié ou éthérifié, un aminoalcoyle inférieur libre ou acylé, un cycloalcoyle en $C_5$ ou $C_6$, un cycloalcoylalcoyle inférieur dont le radical cycloalcoyle contient 5 ou 6 atomes de carbone, un aryle ou un aralcoyle éventuellement substitué, un hétérocyclyle ou hétérocyclylalcoyle inférieur contenant de l'azote, $R_7$ et $R_8$ représentent également ensemble un alcoylène en $C_3$ ou $C_4$ et les radicaux $R_{10}$, $R_{11}$ et $R_{12}$ représentent indépendamment l'un de l'autre un radical carboxy éventuellement estérifié ou amidé et $R_{11}$ représente également un hydrogène, et les sels de tels composés avec des groupes salificateurs, caractérisé en ce qu'on condense de façon connue un composé de formule

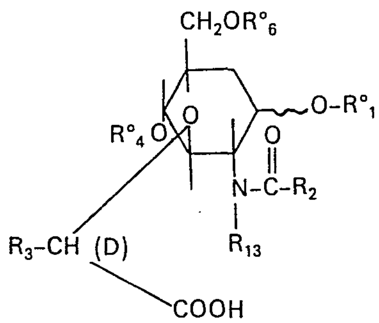

(II)

où $R_2$, $R_3$ et $R_{13}$ ont la signification donnée ci-dessus et $R°_1$, $R°_4$ et $R°_6$ représentent les radicaux $R_1$, $R_4$ ou selon les cas $R_6$ ou un groupe protecteur facilement séparable, ou un dérivé de ce corps avec un composé de formule

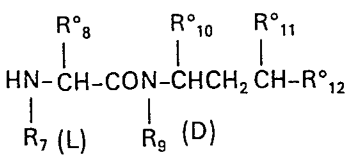

(III)

où $R_7$ et $R_9$ ont la significantion donnée ci-dessus et $R°_8$, $R°_{10}$, $R°_{11}$ et $R°_{12}$ ont la signification de $R_8$, $R_{10}$, $R_{11}$ et $R_{12}$, avec la précision que les groupes carboxy et, si on le désire, les groupes hydroxyle libres présents dans ces radicaux sont protégés par des groupes protecteurs facilement séparables et en ce qu'on sépare les groupes protecteurs éventuellement présents, ou en ce que de façon connue on condense un composé de formule IV

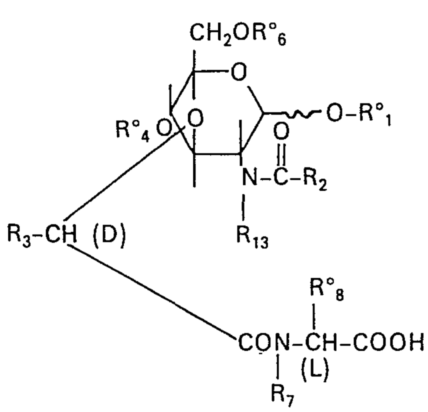

(IV)

où les substituants ont la signification donnée ci-dessus, avec un composé de formule

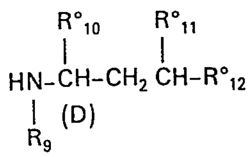

(V)

où $R_9$, $R°_{10}$, $R°_{11}$ et $R°_{12}$ ont la signification donnée ci-dessus, avec la précision que les groupes carboxyle et, si on le désire, les groupes hydroxy libres présents sont protégés par des groupes protecteurs éventuellement présents, ou en ce qu'on fait réagir un composé de formule

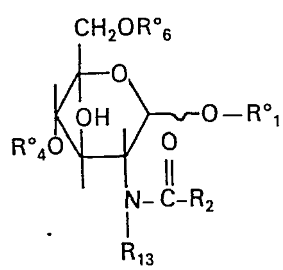

(VI)

où $R_2$, $R°_1$, $R°_4$, $R°_6$ et $R_{13}$ ont les significations données ci-dessus, et les groupes hydroxy qui y sont éventuellement présents sont protégés par un groupe protecteur facilement séparable, avec un composé de formule

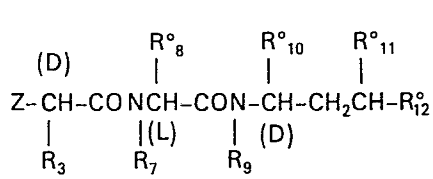

(VII)

où Z représente un groupe hydroxy estérifié réactif et les autres substituants ont la signification donnée ci-dessus, et en ce qu'on sépare les groupes protecteurs éventuellement présents, où les groupes protecteurs facilement séparables sont ceux qui sont connus dans la chimie des peptides ou selon les cas des sucres.